# EUROPEAN PATENT APPLICATION

(11) **EP 1 327 945 A1**
(43) Date of publication of application: **16.07.2003**
(21) Application number: 01956944.1
(22) Date of filing: 17.08.2001
(51) Int. Cl.: G06F 17/60

(54) **REMOTE SERVICE PROVIDING SYSTEM, AND CHARGE COMPUTING METHOD**

(30) Priority: 18.08.2000 JP 2000248535
(71) Applicant: ARKRAY, Inc., Kyoto-shi, Kyoto 601-8045 (JP)
(72) Inventor: DOI, Shigeru, ARKRAY, INC., Kyoto-shi, Kyoto 601-8045 (JP)
(74) Representative: Piésold, Alexander J.
(86) International application number: JP0107086
(87) International publication number: WO02017171

(57) **Abstract**

A remote service providing system that computes a charge according to the frequency of use of the system by each user by connecting the user and a service provider via a communications line. A host computer (1) of a medical institution (A) stores, as a use state of the system by each patient, the number of times of transmission of measurement data from a personal measurement instrument (3) via a communications device (2) and the number of times that the host computer (1) sent instructive messages of a doctor. A computer (5) of a system manager (D) computes a charge to the patient or the medical institution, on the basis of the system use state acquired from the host computer (1) via the communications line (10).

## Description

### Technical Field

The present invention relates to a remote service providing system that, by connecting a measurement device owned by a user such as a patient to a computer of a service provider such as a medical institution via a communications line, enables measurement data of each user to be transmitted to the computer of the service provider so as to manage, and also provide remotely via the communications line a service such as a diagnosis and advice from a doctor or the like on the basis of the measurement data.

### Background Art

Conventionally, personal measurement devices (e.g. electrocardiographs, sphygmomanometers, blood glucose level meters, and urine analysis devices) are known with which a patient can measure various personal data outside of medical institutions.

For example, diabetics, who must regulate their blood glucose level daily, use such a personal measurement device (blood glucose level meter) to measure their blood glucose level four times a day (before or after breakfast, lunch, and dinner, and before going to sleep), and record the results of those measurements in a medical records book or the like. Then, upon visiting a hospital once a month or after longer intervals, patients present their doctor with measurements recorded up to that date and are advised regarding management of their blood glucose level.

However, there have been problems that patients living in remote areas may have difficulties visiting the hospital and that, in the case where patients are supposed to write down the measurement data for themselves, the patients may neglect it.

Accordingly, a system is known in which a personal measurement device is capable of connecting to a server of a medical institution or the like via a public communications network, such as a telephone network, and each time a patient performs a measurement, the measurement data are transmitted to the server from the personal measurement device, so that accurate measurement data can be gathered at the medical institution. If necessary, diagnoses and guidance of doctors based on the collected measurement data are transmitted from the server to the personal measurement device of the patient so that the patient can receive medical services while at home. This so-called remote medical system has been proposed and already has come into practical use in some regions or districts.

In general, the above-described conventional remote medical system is managed by a third institution as a system manager other than the medical institution, and the rate system is fixed. The numbers of services a patient can receive a month is limited in general, i.e., each patient can be advised by the doctor at predetermined numbers of occasions per month

However, since the charge rate is fixed, a patient must pay a predetermined charge even when the patient does not use the service at all in the month. In that case, the patient will feel inevitably that the service is expensive. Furthermore, since the occasions that patients can receive the service per month are limited, sufficient services may not be provided when the patients require.

In order to solve the foregoing problems, it is an object of the present invention to provide a remote guidance system that prevents system users or the service provider (a medical institution or the like) from having a sense of unfairness about the charge and enables sufficient services to be provided to the system users when required, which will be realized by enabling the system managers to compute a charge in accordance with the frequency that each system user uses the system.

### Disclosure of Invention

For achieving the above-described objects, a remote service providing system according to the present invention is characterized in that, by connecting a measurement device owned by a system user and a service providing device of a service provider via a communications line, it transmits measurement data obtained by the measurement device to the service providing device via the communications line and provides a service from the service providing device to the system user via the communications line, wherein the service providing device is provided with a system use information storage portion for storing system use information including a system use state of each system user. The remote service providing system includes a management device having a communications portion connected to the service providing device via the communications line and acquiring from the service providing device the system use information via the communications line, and a charge information processing portion for computing a charge on the basis of the system use state included in the system use information acquired through the communications portion.

According to this configuration, the management device acquires via the communications line the system use information stored in the system use information storage portion of the service providing device, and computes a charge on the basis of a system use state included in the acquired system use information. Here, the charge denotes a sum of money to be charged to at least either a system user or the service provider. Namely, a provider (manager) of this system can charge to the system user as an end user in accordance with the frequency of service use via this system, or it can charge to the service provider making a profit by providing the service via the system, in accordance with the profit provided by the system.

As described above, since the charge is set in accordance with the frequency of use of the service, it is possible to set a proper charge that will not make the system user or the service provider (a medical institution or the like) feel the charge as expensive even when the number of uses of the service is fewer, in comparison with a conventional flat rate system. Moreover, unlike the conventional flat rate system, the number of uses of the system will not be limited, and thus system users can receive required services as they require. As a result, the present invention can provide a remote service providing system that can provide an optimum service to each system user.

In the remote service providing system, it is preferable that the system use state includes the number of receptions of measurement data from a measurement device of each system user.

According to this configuration, a charge to each system user or the service provider is computed in accordance with the number of transmissions of measurement data to the service providing device, and thus it is possible to set a proper charge that will not cause a sense of unfairness among system users and the service provider.

In the remote service providing system, it is preferable that the system use state includes the number of provisions of a remote service to each system user.

According to this configuration, a charge to each system user or the service provider is computed in accordance with the number of times that each system user receives services from the service providing device. Accordingly, a system user will be charged in accordance with the frequency of using the remote service providing system, while a service provider will be charged in accordance with a profit obtained from the service via this remote service providing system, and thus it is possible to set a more proper charge to the system user and to the service provider.

In the remote service providing system, it is preferable that the remote service includes transmission of an instructive message from a doctor or an adviser via the communications line.

According to this configuration, an instructive message from a doctor or an adviser is transmitted via a communications line, and thus a system user can receive services such as advice from a doctor or an adviser while at home. Accordingly, the present invention can provide a remote service providing system that can provide a sufficient service to a system user at a proper fee.

In the remote service providing system, it is preferable that the remote service is a medical service, and the charge information processing portion computes the charge, depending on application of insurance to the medical service.

According to this configuration, when insurance can be applied to the medical service provided as a remote service, a charge corresponding thereto can be computed.

In the remote service providing system, it is preferable that the system use information further includes measurement device information for identifying the category of the measurement item owned by each system user, and the charge information processing portion computes the charge on the basis of the system use state of each system user and the category of the measurement item owned by the system user.

According to this configuration, it is possible to compute a proper charge corresponding to the category of the measurement item.

In the remote service providing system, it is preferable that the management device transmits, through the communications portion, the charge computed by the charge information processing portion to the service providing device, and the service providing device includes the charge information storage portion that stores the charge.

According to this configuration, the charge is transmitted to the service providing device, and thus the service provider can keep track of the charge to each system user. Thereby for example, when the service provider is a medical institution, this information can be used for charging a doctor's fee or the like.

For achieving the above-described objects, a service providing device according to the present invention is a service providing device for a remote service providing system, and, by connecting a measurement device owned by a system user and a service providing device of a service provider via a communications line, the remote service providing system transmits measurement data obtained by the measurement device to the service providing device via the communications line and provides a remote service from the service providing device to the system user via the communications line, wherein the remote service providing system is provided with a management device for computing a charge on the basis of a system use state of each system user, and it includes a system use information storage portion for storing system use information including a system use state of each system user and a communications portion for transmitting the system use information of each system user to the management device via a communications line.

According to this configuration, in a service providing device, system use information including a system use state of each system user is stored in the system use information storage portion, which is transmitted through the communications portion to the management device of the remote service providing system, and in the management device, a charge to each system user or the service provider is computed on the basis of this system use information.

Accordingly, the charge is set in accordance with the frequency of use of the service by each system user, and thus it is possible to compute a proper charge such that the system user or the service provider will not consider it expensive even when the number of service uses is fewer, in comparison with a conventional flat rate system. Moreover, unlike the conventional system, the number of uses of the system will not be limited, and thus system users can receive any required services as they require. As a result, the present invention can provide a service providing device that can provide an optimum service to each system user.

In the service providing device, it is preferable that the communications portion receives the charge computed by the management device of the remote service providing system from the management device via the communications line, and the service providing device has a charge information storage portion for storing the charge received through the communications portion.

According to this configuration, a charge to each system user or the service provider is transmitted from the management device to the service providing device, and thus the service provider can keep track of the charge to each system user. Thereby for example, when the service provider is a medical institution, this information can be used for charging a doctor's fee or the like.

In the service providing device, it is preferable that the system use state includes the number of receptions of measurement data from a measurement device of each system user.

According to this configuration, the charge to the system user or the service provider will be computed in accordance with the number of transmissions of measurement data to the service providing device, thereby enabling a proper charge to be set that will not cause any sense of unfairness among system users and the service provider.

In the service providing device, it is preferable that the system use state includes the number of provisions of a remote service to each system user.

According to this configuration, a charge to each system user or the service provider is computed in accordance with the number of times that each system user receives a remote service from the service providing device. Accordingly, the system user is charged in accordance with the frequency of using this remote service providing system, and the service provider is charged in accordance with a profit obtained from the remote service provided through this remote service providing system, and thus it is possible to set a more proper charge for the system user and the service provider.

In the service providing device, it is preferable that the remote service includes transmission of an instructive message from a doctor or adviser via the communications line.

According to this configuration, an instructive message from a doctor or an adviser is transmitted via the communications line, and thus a system user can receive a remote service such as advice from a doctor or an adviser while at home or the like. Accordingly, the present invention can provide a remote service providing system that can provide a sufficient remote service to the system user at a proper fee.

For achieving the above-described objects, a management device according to the present invention is a management device for a remote service providing system, and, by connecting a measurement device owned by a system user and a service providing device of a service provider via a communications line, the remote service providing system transmits measurement data obtained through the measurement device to the service providing device via the communications line and provides a remote service from the service providing device to the system user via the communications line, and the management device has a communications portion for acquiring system use information including a system use state of each system user from the service providing device via the communications line and a charge information processing portion for computing a charge on the basis of the system use state included in the system use information acquired through the communications portion.

According to this configuration, the management device acquires, from the service providing device, system use information including a system use state of each system user via the communications line, and computes a charge to each system user or the service provider on the basis of the acquired system use information.

Thereby, a charge is set in accordance with the frequency of use of the service for each system user while a charge to the service provider is set in accordance with a profit obtained from providing services through this system, and thus either the system user or the service provider will not consider it expensive even when the frequency of using the system is fewer, in comparison with a conventional flat rate system. Moreover, unlike the conventional system, the number of uses of the system will not be limited, and thus system users can receive any required services as they require. As a result, the present invention can provide a remote service providing system that can provide an optimum service to each system user.

In the management device of the remote service providing system, it is preferable that the communications portion transmits the charge computed by the charge information processing portion to the service providing device.

According to this configuration, a medical institution can keep track of, for example, a charge to each system user, and thus this information can be used for requesting a doctor's fee or the like.

In the management device of the remote service providing system, it is preferable that the system use state includes the number of receptions of measurement data from a measurement device of each system user.

According to this configuration, a charge to each system user or the service provider will be computed in accordance with the number of transmissions of measurement data to the service providing device, and thus it is possible to set a proper charge that will not cause any sense of unfairness among system users and the service provider.

In the management device of the remote service providing system, it is preferable that the system use state includes the number of times of provisions of a remote service to each system user.

According to this configuration, a charge to each system user or the service provider is computed in accordance with the number that each system user receives a remote service from the service providing device. Accordingly, the system user is charged in accordance with the frequency of using this remote service providing system, and the service provider is charged in accordance with a profit obtained from the remote service via this remote service providing system, and thus it is possible to set a more proper charge for the system user and the service provider.

In the management device of the remote service providing system, it is preferable that the remote service includes transmission of an instructive message from a doctor or an adviser via the communications line.

According to this configuration, an instructive message from a doctor or an adviser is transmitted via the communications line, and thus a system user can receive a remote service such as advice from a doctor or an adviser while at home or the like. Accordingly, the present invention can provide a remote service providing system that can provide a sufficient remote service to the system user at a proper fee.

For achieving the above-described objects, a charge computing method according to the present invention includes, by connecting a measurement device owned by a system user and a service providing device of a service provider via a communications line, transmission of measurement data obtained by the measurement device to the service providing device via the communications line and also providing a remote service from a service providing device to a system user via the communications line, and the method includes: in the service providing device, storing system use information including a system use state of each system user; acquiring from the service providing device the system use information for each system user; and computing a charge on the basis of a system use state included in the acquired system use information.

According to this configuration, system use information including a system use state of each system user is acquired from the service providing device via the communications line, and a charge to each system user or the service provider is computed on the basis of the acquired system use information.

Accordingly, a charge is set in accordance with the frequency of use of the service, and thus it is possible to set a proper charge such that the system user or the service provider will not consider it expensive even when the number of uses of the service is fewer, in comparison with a conventional flat rate system. Moreover, unlike the conventional system, the number of uses of the system will not be limited, and thus system users can receive required services as they require. As a result, the present invention can provide a remote service providing system that can provide an optimum service to each system user.

In the charge computing method in the remote service providing system, it is preferable that that the system use state includes the number of receptions of measurement data from a measurement device of each system user.

According to the method, a charge to each system user or the service provider will be computed in accordance with the number of transmissions of measurement data to the service providing device, and thus it is possible to set a proper charge that will not cause any sense of unfairness among system users and the service provider.

In the charge computing method in the remote service providing system, it is preferable that the system use state includes the number of provisions of a remote service to each system user.

According to this configuration, a charge to each system user or the service provider is computed in accordance with the number that each system user receives a remote service from the service providing device. Accordingly, the system user is charged in accordance with the frequency of using this remote service providing system, and the service provider is charged in accordance with a profit obtained from the remote service via this remote service providing system, and thus it is possible to set a more proper charge for the system user and the service provider.

### Brief Description of Drawings

FIG. 1 is a block diagram showing the configuration of a remote medical system according to one embodiment of the present invention.
FIG. 2 is a block diagram showing the configuration of a personal measurement instrument included in the remote medical system.
FIG. 3 is a block diagram showing the configuration of a communications device included in the remote medical system.
FIG. 4 is a block diagram showing the configuration of a host computer for a medical institution, which is included in the remote medical system.
FIG. 5 is a block diagram showing the configuration of a computer of a system manager, which is included in the remote medical system.
FIG. 6 is a flow chart showing the operation of the personal measurement instrument.
FIG. 7 is a flow chart showing the operation of the communications device.
FIG. 8 is a flow chart showing the operation of the host computer of the medical institution.
FIG. 9 is a flow chart showing the operation of the computer of the system manager.

### Best Mode for Carrying out the Invention

### (First Embodiment)

Hereinafter, a remote medical system as an embodiment of the remote service providing system of the present invention is described with reference to the drawings.

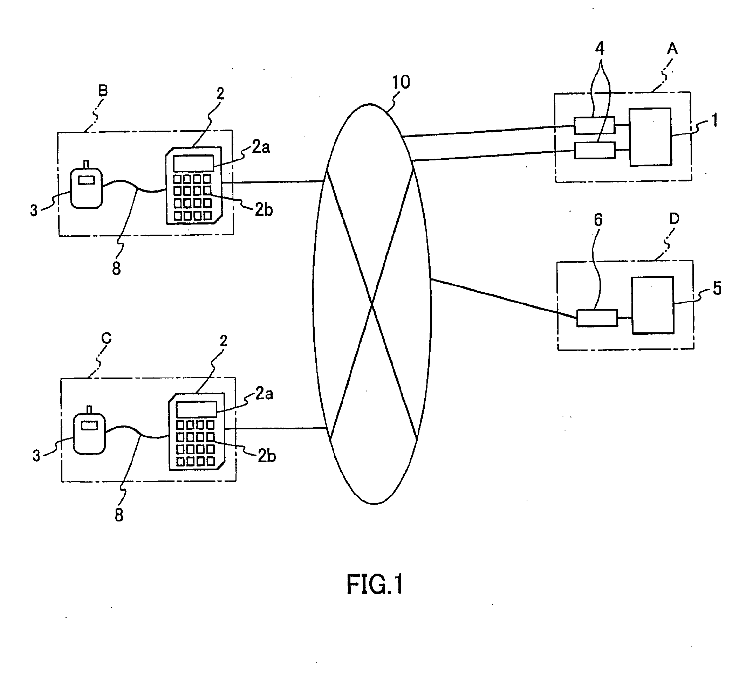

FIG. 1 is a block diagram schematically showing the configuration of a remote medical system according to an embodiment of the present invention. As shown in FIG. 1, the remote medical system is configured so that a host computer 1 of a medical institution A can be connected to each household B, C, etc. of a patient via DCE (data circuit terminating equipment) 4 and a communications line 10 such as a telephone network.

In each household B, C, etc. of a patient, a personal measurement instrument 3, which the patient uses for self-measurement, is connected to a communications device 2 via a communications cable 8. The communications device 2 is connected to the medical institution A via the communications line 10, and executes transmission and reception of data or the like with the host computer 1 via the DCE 4.

The patient possessing the personal measurement instrument 3 performs measurement by using the personal measurement instrument 3 at home or the like, and transmits the measurement data to the medical institution A by using the communications device 2. Transmission of the measurement data can be executed at every measurement or at a predetermined timing (e.g., once a day or a week). Alternatively, measurement results can be transmitted in a batch after performing a predetermined number of cycles of measurement.

The host computer 1 receives and stores data transmitted from the personal measurement instrument 3 of the patient so as to be available for reference when the patient comes to see a doctor. If necessary, the doctor advises the patient via the communications line 10 on the basis of the data transmitted from the patient.

In addition to that, a computer 5 of a system manager D of the remote medical system is connected to the communications line 10 via the DCE 6. This computer 5 is connected to the host computer 1 of the medical institution A via the communications line 10.

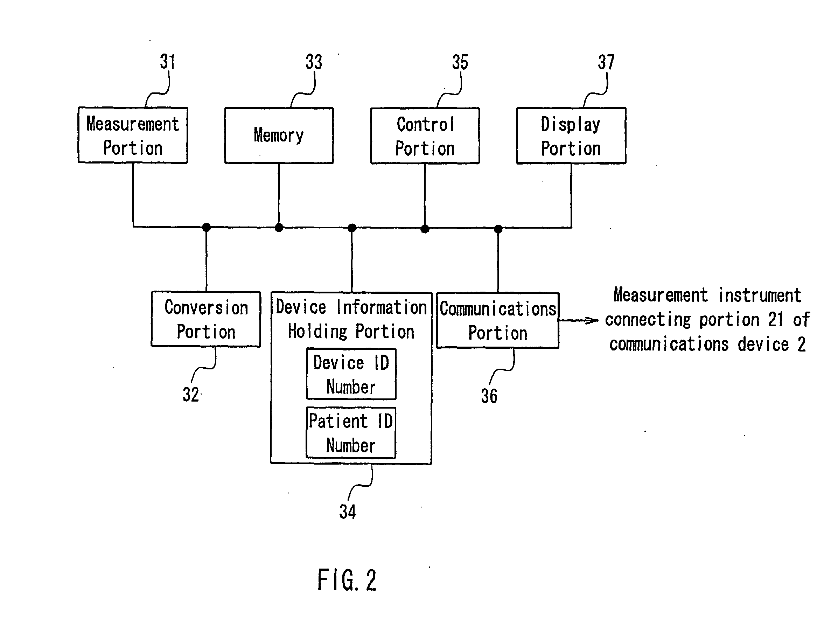

The configuration of the personal measurement instrument 3 is described below. FIG. 2 is a block diagram showing the configuration of the personal measurement instrument 3. As shown in FIG. 2, the personal measurement instrument 3 includes a measurement portion 31, a conversion portion 32, a memory 33, a device information holding portion 34, a control portion 35, a communications portion 36, and a display portion 37.

The measurement portion 31 has a sensor that corresponds to the function of the personal measurement instrument 3, and measures data in accordance with a control by the control portion 35. For example, when the personal measurement instrument 3 is a blood glucose level meter, it measures a blood glucose value electrically or optically from a specimen to which a small amount of blood collected from a patient's fingertip or the like adheres.

The conversion portion 32, in accordance with a control by the control portion 35, determines the concentration of a measured substance by executing a process such as IV conversion or AD conversion in response to information obtained by the measurement portion 31, and stores this information in the memory 33 as measurement data.

The device information holding portion 34 stores a device ID number allocated uniquely to each personal measurement instrument 3 and a patient ID number allocated to a patient possessing the personal measurement instrument 3. The control portion 35 controls operations of the respective portions of the personal measurement instrument 3. The communications portion 36 is connected to the communications device 2 via a cable 8, and transmits measurement data or the like to the communications device 2 in accordance with a control by the control portion 35. The display portion 37 has a liquid crystal display or the like and displays a message or the like.

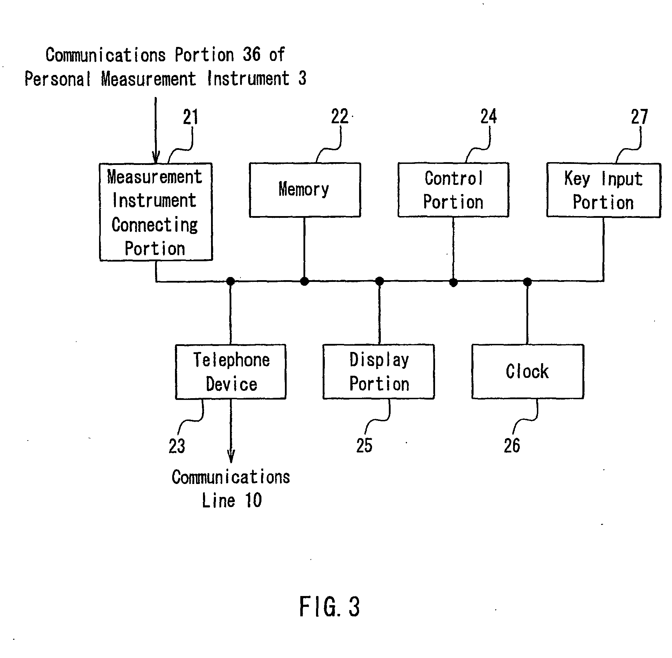

The communications device 2 has a configuration as described below. As shown in FIG. 1, the communications device 2 has a liquid crystal display 2a and an input key 2b that can input figures, letters or the like. FIG. 3 is a block diagram showing the configuration of the communications device 2. As shown in FIG. 3, the communications device 2 includes a measurement instrument connecting portion 21, a memory 22, a telephone device 23, a control portion 24, a display portion 25, a clock 26 and a key input portion 27.

The measurement instrument connecting portion 21 is connected to a communications portion 36 of a personal measurement instrument 3, and receives measurement data or the like from the personal measurement instrument 3. The memory 22 stores measurement data or the like that the measurement instrument connecting portion 21 received from the personal measurement instrument 3. The telephone device 23 dials for a connection to the host computer 1 of the medical institution A via the communications line 10, and establishes a connection so as to transmit and receive data.

The control portion 24 controls operations of the respective portions of the communications device 2. The display portion 25 controls the liquid crystal display 2a and displays various messages or the like. The key input portion 27 receives operations of the input key 2b by the patient. That is, the patient can direct execution of various operations such as transmission of measurement data, to the communications device 2.

The communications device 2 can be formed as a mobile telephone or a PHS that can be programmed. This embodiment shows a configuration in which a communications device 2 and a personal measurement instrument 3, both of which are separate and independent devices, are connected to each other via the cable 8. Alternatively, the communications device 2 and the personal measurement instrument 3 can be formed integrally. The device information holding portion 34 shown in FIG. 2 can be provided to the communications device 2, or the key input portion 27 shown in FIG. 3 can be provided to the personal measurement instrument 3.

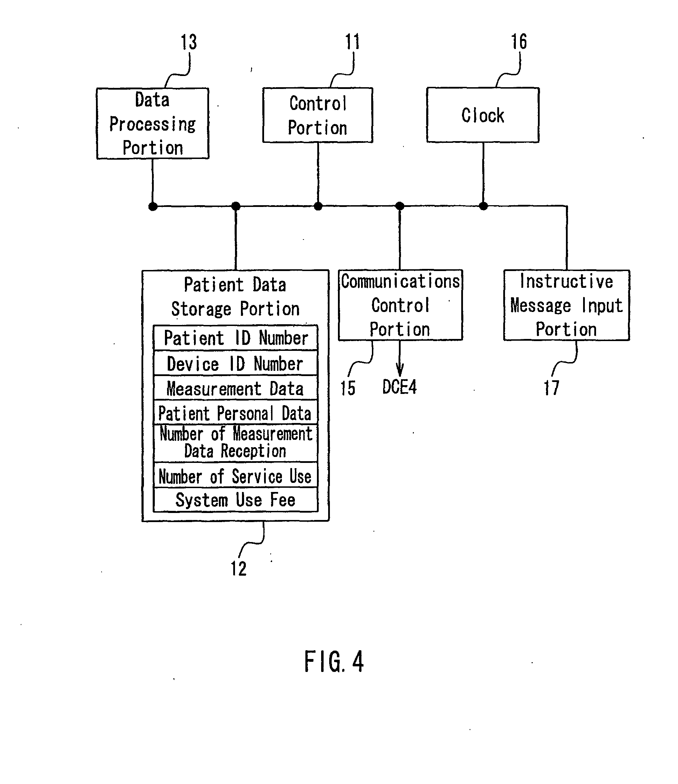

The following description is about the configuration of the host computer 1 of the medical institution A. FIG. 4 is a block diagram showing the functional configuration of the host computer 1. As shown in FIG. 4, the host computer 1 includes a control portion 11, a patient data storage portion 12, a data processing portion 13, a system use information storage portion 14, a communications control portion 15, a clock 16, an instructive message input portion 17, and a charge information storage portion 18.

The control portion 11 controls operations of the respective portions of the host computer 1. The communications control portion 15 transmits and receives data or the like with the communications device 2 at the household of the patient. The data processing portion 13 corrects measurement data transmitted from the patient and executes various computations such as determining whether the values of the corrected data is within a normal range.

The patient data storage portion 12 stores personal information of each patient, such as a patient ID number uniquely assigned to the patient, a device ID number uniquely assigned to the personal measurement instrument 3 owned by the patient, measurement data transmitted from the patient, and the name and address of the patient. The system use information storage portion 14 stores the number of measurement data receptions and the number of service uses. The charge information storage portion 18 stores the system use fee (charge).

The patient ID number denotes a number applied uniquely to each patient. The device ID number denotes a number applied uniquely to a personal measurement instrument 3 owned by each patient. The number of measurement data receptions denotes the number of times that each patient transmits measurement data. The number of service use denotes the number of times that each patient is advised by a doctor of the medical institution A via this remote medical system. As described below, these data are used for computing the system use fee of each patient.

The instructive message input portion 17 is provided for inputting a message that the doctor sends to a patient via the communications line 10 when the doctor dispenses advice or the like on the basis of the measurement data transmitted from the patient.

Though the patient data storage portion 12, the system use information storage portion 14, and the charge information storage portion 18 are shown as independent blocks in this embodiment, these storage portions can be located on a physically-identical memory. Alternatively, some of the functional blocks shown in FIG. 4 can be configured as peripheral equipment of the host computer 1. For example, the patient data storage portion 12, the system use information storage portion 14 and the charge information storage portion 18 can be located in a disk device, a file server or the like to be connected to the host computer 1 via LAN or the like. Alternatively, the instructive message input portion 17 can be located at any other computer terminals or the like in the medical institution A similarly connected to the host computer 1 via LAN or the like.

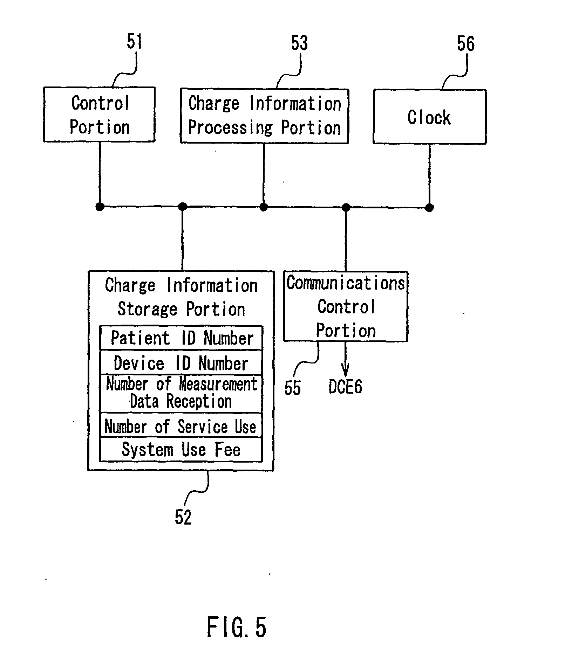

The next description is about the configuration of the computer 5 of the system manager D of this remote medical system. FIG. 5 is a block diagram showing a functional configuration of the computer 5. As shown in FIG. 5, the computer 5 has a control portion 51, a charge information storage portion 52, a charge information processing portion 53, a communications control portion 55, and a dock 56.

The control portion 51 controls operations of the respective portions of the computer 5. The communications control portion 55 is connected to the host computer 1 of the medical institution A via the DCE 6 and the communications line 10 and receives, from the host computer 1, information (system use information) about a system use state of each patient. This information is used for computing a system use fee for each patient, and specifically, it includes the patient ID number and the device ID number stored in the patient data storage portion 12 of the host computer 1, and the number of measurement data receptions and the number of service uses stored in the system use information storage portion 14. These pieces of information received by the communications control portion 55 are stored in the charge information storage portion 52.

The charge information processing portion 53 computes a system use fee for each patient on the basis of the above-described information obtained from the host computer 1 of the medical institution A. The computed system use fee is stored temporarily in the charge information storage portion 52 and transmitted to the host computer 1 at a suitable timing.

Regarding the configuration of the computer 5, similar to the configuration of the host computer 1, the charge information storage portion 52 or the like can be provided as the peripheral equipment for the computer 5.

The operations of this remote medical system will be detailed below.

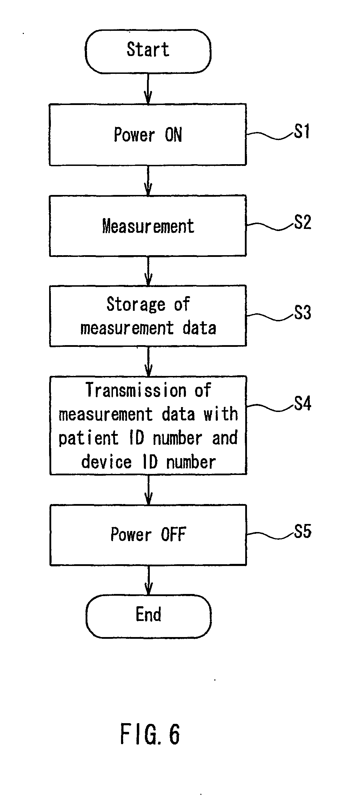

First, the operations of the personal measurement instrument 3 are described in reference to FIG. 6.

When a patient performs a measurement, first, a power source of the personal measurement instrument 3 is turned on (step S1). Next, measurement is performed through procedures corresponding to the test substance and measurement items (step S2). As described above, when the personal measurement instrument 3 is a blood glucose level meter, a small amount of blood that the patient collects by pricking his fingertip or the like is used for the test substance, and this blood is put into a sensor of the measurement portion 31 so as to measure the blood glucose level optically or electrically.

The control portion 35 sends the information obtained at the measurement portion 31 to the conversion portion 32 so as to convert it to measurement data as numeric data. The thus obtained measurement data are stored temporarily in the memory 33 (step S3).

Then, in accordance with a control by the control portion 35, the communications portion 36 establishes a connection to the measurement instrument connecting portion 21 of the communications device 2, reads measurement data that have been stored temporarily in the memory 33 and transmits to the communications device 2 (step S4). At this time, the device ID number and the patient ID number stored in the device information holding portion 34 are sent to the communications device 2 together with the above-described measurement data.

When transmission of the measurement data or the like is completed, the power source of the personal measurement instrument is 3 turned off (step S5). The power source can be turned off manually by the patient, or it can be configured to turn off automatically after a predetermined time without any particular operations.

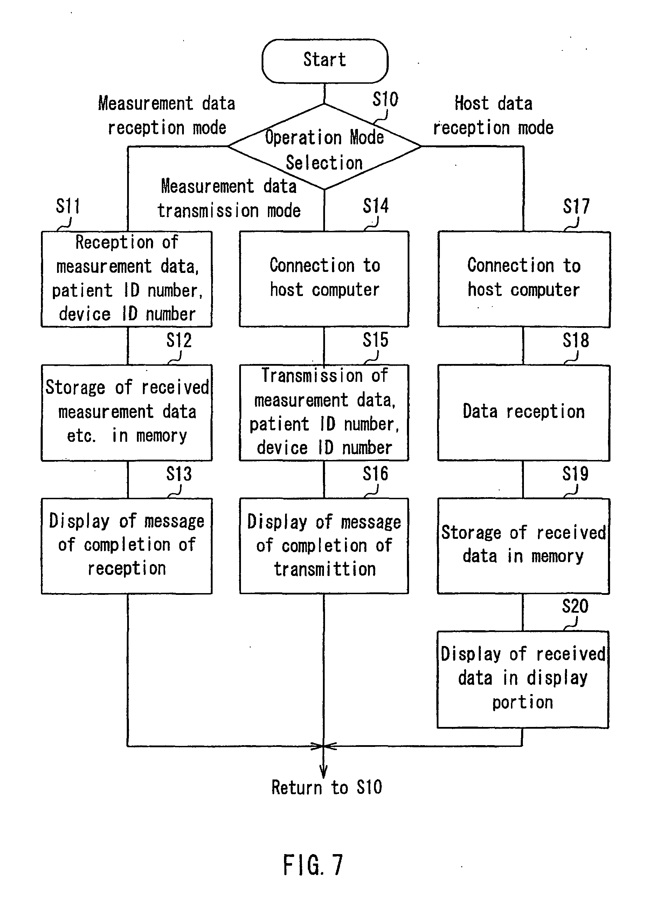

Next, the operations of the communications device 2 are described below with reference to FIG. 7.

The communications device 2 has a plurality of operation modes. In this embodiment, the following description is based on a premise that the communications device 2 has three categories of operation modes, i.e., "a measurement data reception mode" for receiving measurement data from the personal measurement instrument 3, "a measurement data transmission mode" for transmitting the measurement data received from the personal measurement instrument 3 to the host computer 1 of the medical institution A, and "a host data reception mode" for receiving various data such as advice of a doctor from the host computer 1.

The communications device 2 has a power source that is in an ON state continuously, and the control portion 24 is triggered by a predetermined condition such as an external input so as to select any of the above-described three operation modes, and starts the operation. For example, the measurement data reception mode is selected by notifying the start of the data transmission, from the communications portion 36 of the personal measurement instrument 3 to the measurement instrument connecting portion 21 of the communications device 2. The measurement data transmission mode is selected at a timing that is previously determined as a timing to transmit measurement data from the communications device 2 to the host computer 1. The host data reception mode is selected by notifying the start of the data transmission, from the communications control portion 15 of the host computer 1 to the telephone device 23 of the communications device 2.

The procedures for the respective modes are described below.

A first description is about the operations in the measurement data reception mode.

In step S4 shown in the flow chart of FIG. 6, when a data transmission from the communications portion 36 of the personal measurement instrument 3 to the measurement instrument connecting portion 21 of the communications device 2 starts, the control portion 24 of the communications device 2 allows the measurement instrument connecting portion 21 to receive the measurement data transmitted from the personal measurement instrument 3, the patient ID number and the device ID number (step S11 in FIG. 7), and to store in the memory 22 the received measurement data, patient ID number and device ID number (step S12). When the process of step S12 is completed normally, a message that the reception of the measurement data is completed normally is displayed on the display portion (step S13), and the control is returned to the step S10.

The next description is about operations in the measurement data transmission mode.

As described above, the measurement data transmission mode is selected at a timing (hereinafter, referred to as a transmission timing) that is predetermined as a timing for transmitting measurement data from the communications device 2 to the host computer 1, and the process starts.

The transmission timing can be at every time of receiving measurement data from the personal measurement instrument 3, or it can be at a predetermined tuning (e.g., a predetermined time a day, a predetermined time of a predetermined day a week, or a predetermined time of a predetermined day a month) determined on the basis of time information acquired from the clock 26. Alternatively, the transmission timing can be at a time that the number of the measurement data stored in the memory 22 reaches a predetermined level.

At the transmission timing, the control portion 24 of the communications device 2 is connected to the host computer 1 via the communications line 10 and the DCE 4 through the telephone device 23 (step S14), acquires from the memory 22 the measurement data, the device ID number and the patient ID number, and transmits them to the host computer 1 (step S15).

And when the transmission in the step S15 is completed normally a message that transmission of the measurement data is completed normally is displayed on the display portion 25 (step S16), and the control is returned to the step S10.

The last description is about operations in the host data reception mode.

The data transmitted from the host computer 1 to a patient includes mainly a test result based on the measurement data transmitted to the host computer 1, an instructive message from a doctor, or the like.

When a connection request is executed by the communications control portion 15 of the host computer 1 to the telephone device 23 of the communications device 2 via the communications line 10, the telephone device 23 establishes a connection to the host computer 1 (step S17). When a data transmission from the host computer 1 starts, the control portion 24 allows the telephone device 23 (step S18) to receive the data and store the received data in the memory 22 (step S19) and at the same time, displays the data on the display portion 25 (step S20). After the patient confirms the content displayed on the display portion 25, the control portion 24 returns the control to step S10.

In this manner, after the patient transmits measurement data from the communication device 2 to the host computer 1, a test result based on the measurement data and an instructive message from a doctor are sent back from the host computer 1 and displayed on the display portion 25, so that the patient can receive a medical service while at home.

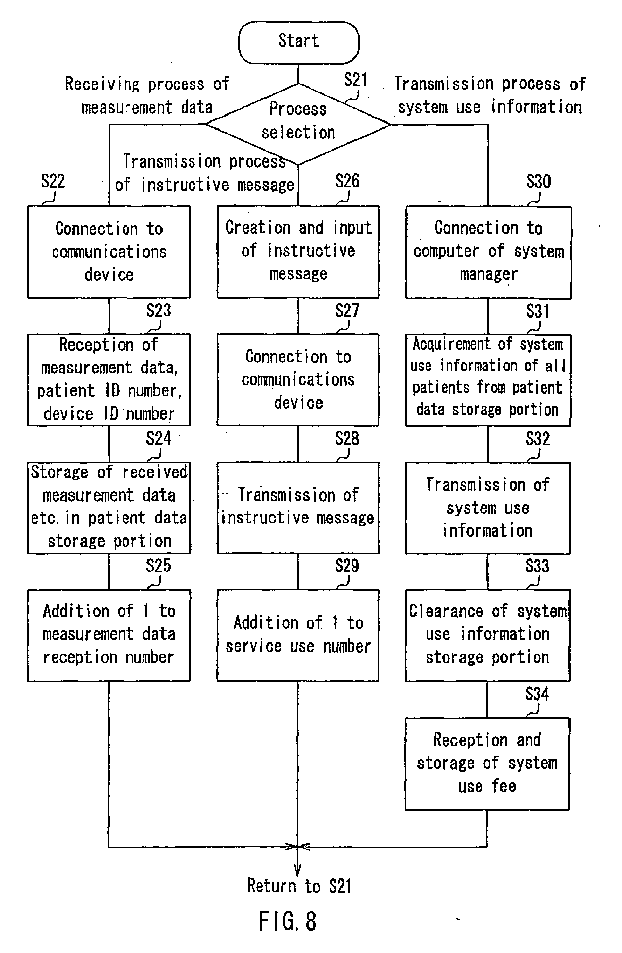

Next, the operations of the host computer 1 of the medical institution A are described below with reference to FIG. 8.

The host computer 1 as a general-purpose computer executes various processes. Here, the description refers only to processes relating directly to the present invention, i.e., processes for receiving measurement data from a patient, transmitting an instructive message or the like from a doctor to a patient, and transmitting system use information of each patient to the computer 5 of the data system manager D.

The process of receiving measurement data from the communications device 2 of the patient starts when a connection for transmitting the measurement data or the like is requested from the communications device 2 (step S14 in FIG. 7). The control portion 11 establishes a connection between the communications control portion 15 and the telephone device 23 of the communications device 2 via the DCE 4 and the communications line 10 (step S22 in FIG. 8), and allows the communications control portion 15 to receive the measurement data and also a patient ID number and a device ID number transmitted together with the measurement data (step S23).

The control portion 11 stores the received measurement data or the like in the patient data storage portion 12 (step S24). At this time, '1' is added to a value of "number of measurement data receptions" of the patient which is stored in the patient data storage portion 12 (step S25).

Processes of transmitting an instructive message or the like from a doctor to a patient are described below.

The instructive message is created and inputted via the instructive message input portion 17 by a doctor of the medical institution A periodically or in correspondence with a request from a patient, by referring to measurement data stored in the patient data storage portion 12 (step S26).

In accordance with a control by the control portion 11, the communications control portion 15 establishes a connection to the communications device 2 via the DEC 4 and the communications line 10 (step S27), and transmits an instructive message inputted by a doctor (step S28). When the transmission of the message is completed normally, the control portion 11 adds '1' to a value of "number of service uses" of the patient, which is stored in the patient data storage portion 12 (step S29).

A process of transmitting system use information of each patient to the computer 5 of the system manager D is as follows. As described later, the computer 5 of the system manager D requests to the host computer 1 of the medical institution A at a predetermined timing (for example, once a day, or once a month) to transmit system use information including a system use state of each patient.

Receiving a data transmission request from the computer 5, the control portion 11 of the host computer 1 establishes on the communications control portion 15 a connection to the computer 5 via the DCE 4 and the communications line 10 (step S30), and retrieves system use information (patient ID number, device ID number, number of measurement data receptions, and number of service uses) of all patients (step S31), and transmits it to the computer 5 (step S32).

When the transmission is completed normally, the control portion 11 clears the values of the number of measurement data receptions and the number of service uses stored in the system use information storage portion 14 (step S33).

As described below, after a transmission of the system use fee to the computer 5, a charge (system use fee) to each patient or to the medical institution is computed at the computer 5 and sent back, and the control portion 11 stores the value in a region for 'system use fee' of the patient data storage portion 12 (step S34).

The transmission of the system use fee of each patient or the medical institution from the computer 5 is not limited to immediately follow the transmission of the system use information from the medical institution to the system manager, but it can be executed by establishing a connection between the computer 5 of the system manager D and the host computer 1 of the medical institution A at any suitable timing.

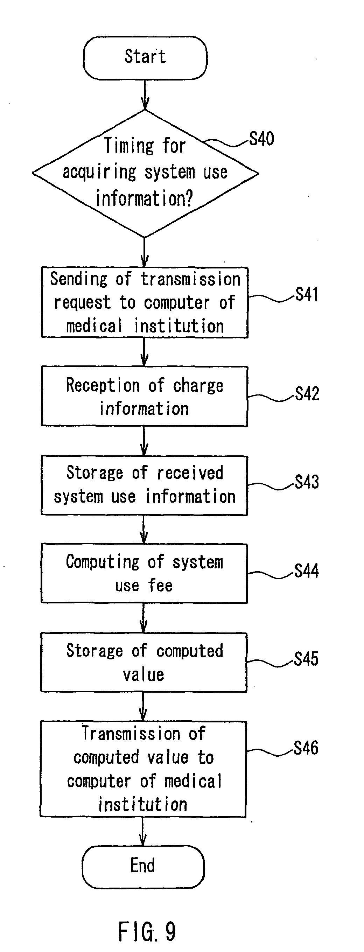

Next, operations of the computer 5 of the system manager D are described with reference to FIG. 9.

The computer 5 of the system manager D acquires system use information of each patient from the host computer 1 of the medical institution A, and referring to this, computes a system use fee of each patient or the medical institution in the following procedures.

The control portion 51 of the computer 5 detects, on the basis of time information obtained from the clock 56, a predetermined timing (e.g., a predetermined time a day, a predetermined time of a predetermined day a week, or a predetermined time of a predetermined day a month) (step S40), and sends, through the communications control portion 55 a data transmission request to the host computer 1 of the medical institution A via the DCE 6 and the communications line 10 (step S41).

Furthermore, as shown in step S32 of FIG. 8, it receives system use information (patient ID number, device ID number, number of measurement data receptions, and number of service uses) transmitted from the host computer 1 (step S42), and stores it in the charge information storage portion 52 (step S43).

Furthermore, in accordance with a control of the control portion 51, the charge information processing portion 53 computes a system use fee of each patient and the medical institution, on the basis of the number of measurement data receptions and the number of service uses stored in the charge information storage portion 52 (step S44), and stores the computed values in the charge information storage portion 52 (step S45), and at the same time, through the communications control portion 55, transmits to the host computer 1 of the medical institution A via the DCE 6 and the communications line 10 (step S46).

Regarding step S44, various methods can be applied for computing the system use fee, on the basis of the number of measurement data receptions and the number of service uses.

For example, the system use fee can be computed by determining previously a fee for a reception of measurement data and a fee for a use of the service, and multiplying them with the number of measurement data receptions and the number of service uses respectively so as to obtain the total.

Alternatively, charging of system use fees varied depending on the categories of the personal measurement instruments 3 can be applied. Furthermore, when a personal measurement instrument 3 can measure plural items of measurements, a separate system use fee can be charged depending on the categories of the measured items. In this case, either a device ID number that can identify the category of the personal measurement instrument 3 or any other information enabling identification of the category of the measured item is transmitted from the communications device 2 to the host computer 1, and based on these values, the computer 5 of the system manager D determines the category of the personal measurement instrument 3 (e.g., a blood glucose level meter, an urine analysis device, and an electrocardiograph) the patient uses or the category to be measured with the personal measurement instrument 3, and multiplying a unit charge corresponding to these categories with the number of measurement data receptions.

Assuming a case that insurance is applied to the provision of the medical service through this system, the system use fee can be computed corresponding to cases in which the insurance is applied or not, by referring to the data of a remuneration for medical services.

In this embodiment, every time the computer 5 of the system manager D receives system use information of each patient from the host computer 1 of the medical institution A, it sends back to the host computer 1 a system use fee of each patient or the medical institution, which is computed on the basis of the system use information. The process of transmitting the system use fee to the host computer 1 can be executed at any suitable timing.

Though in this embodiment the system use fees of the patient and the medical institution are computed at the computer 5, the fees are not necessarily charged on both the patient and the medical institution but a system use fee can be computed for at least either the patient or the medical institution.

Furthermore, according to the structural example of the medical service providing device (FIG. 4) in this embodiment, the number of measurement data receptions and the number of service uses that represent the system use state are stored in the system use information storage portion 14. The system use information regarding the present invention is not limited only to the number of measurement data receptions and the number of service uses, but it can include any arbitrary information required for computing charges, for example, various information stored in the patient data storage portion 12 and any other information.

Though in this embodiment a host computer is described as an example of a medical service providing device located in a medical institution, this is not limitative and can be replaced by a work station, a personal computer or the like.

Furthermore in this embodiment, transmission of an instructive message from a doctor is described as an example of a medical service counted as the number of service uses, and it is also possible to include in the medical service to be counted, for example, transmission of a result of analysis of the measurement data by means of a computer of a medical service.

Though in this embodiment an instructive message from a doctor of a medical institution is displayed on a display portion of a communications device owned by a patient, provision of medical services will not be limited thereto. The configuration can be, for example, transmission of an instructive message or the like from a doctor to a telephone device or a facsimile device of a household of a patient or to an e-mail address on an internet the patient has.

Furthermore, though a medical service is described as an example of remote services in this embodiment, the field to apply the present invention will not be limited to medical purposes. The present invention can be used, for example, for a training-support system in which a heart rate and data of a pedometer are transmitted from a measurement device of a user to a management device, and an adviser checks remotely the transmitted data and transmits advice to the user.

### Industrial Applicability

As described above, the present invention is applied to a remote service providing system that connects a system user and a service provider via a communications line, and the present invention can provide a remote service providing system in which a computer of a system manager acquires, from a computer of the service provider, information relating to a system use state of each system user and computes a charge on the basis of the information so as to provide sufficient services as system users require, without causing any sense of unfairness about the charge for the system users.

## Claims

1. A remote service providing system that, by connecting a measurement device owned by a system user and a service providing device of a service provider via a communications line, transmits measurement data obtained by the measurement device to the service providing device via the communications line and provides a service from the service providing device to the system user via the communications line, wherein
the service providing device is provided with a system use information storage portion for storing system use information comprising a system use state of each system user; and
the remote service providing system comprises a management device having a communications portion connected to the service providing device via the communications line and acquiring from the service providing device the system use information via the communications line, and a charge information processing portion for computing a charge on the basis of the system use state included in the system use information acquired from the communications portion.

2. The remote service providing system according to claim 1, wherein the system use state comprises the number of measurement data receptions from a measurement device of each system user.

3. The remote service providing system according to claim 1 or 2, wherein the system use state comprises the number of provisions of a remote service to each system user.

4. The remote service providing system according to claim 3, wherein the remote service comprises transmission of an instructive message from a doctor or an adviser via the communications line.

5. The remote service providing system according to claim 3 or 4, wherein the remote service is a medical service, and the charge information processing portion computes the charge, depending on application of insurance to the medical service.

6. The remote service providing system according to any one of claims 1 to 5, wherein the system use information further comprises measurement device information for identifying a category of a measurement item of each system user, and
the charge information processing portion computes the charge on the basis of the system use state of each system user and the category of the measurement item of the system user.

7. The remote service providing system according to any one of claims 1 to 6, wherein the management device transmits through the communications portion to the service providing device the charge computed by the charge information processing portion, and the service providing device comprises a charge information storage portion for storing the charge.

8. A service providing device in a remote service providing system,
the remote service providing system, by connecting a measurement device owned by a system user and a service providing device of a service provider via a communications line, transmits measurement data of the measurement device to the service providing device via the communications line and provides a service from the service providing device to the system user via the communications line, wherein
the remote service providing system is provided with a management device for computing a charge on the basis of a system use state of each system user, and
the service providing device comprises a system use information storage portion for storing system use information including a system use state of each system user, and a communications portion for transmitting the system use information of each system user to the management device via a communications line.

9. The service providing device according to claim 8, wherein the communications portion receives the charge computed by the management device of the remote service providing system from the' management device via the communications line, and
the service providing device comprises a charge information storage portion for storing the charge received through the communications portion.

10. The service providing device according to claim 8 or 9, wherein the system use state comprises the number of measurement data receptions from a measurement device of each system user.

11. The service providing device according to any one of claims 8 to 10, wherein the system use state comprises the number of provisions of a remote service to each system user.

12. The service providing device according to claim 11, wherein the remote service comprises transmission of an instructive message from a doctor or an adviser via the communications line.

13. A management device of a remote service providing system, the remote service providing system, by connecting a measurement device owned by a system user and a service providing device of a service provider via a communications line, transmitting measurement data obtained from the measurement device to the service providing device via the communications line and provides a remote service from the service providing device to the system user via the communications line, the remote management device comprising:
a communications portion for acquiring system use information comprising a system use state of each system user from the service providing device via the communications line; and
a charge information processing portion for computing a charge on the basis of the system use state included in the system use information acquired through the communications portion.

14. The management device of the remote service providing system according to claim 13, wherein the communications portion transmits the charge computed by the charge information processing portion to the service providing device.

15. The management device of the remote service providing system according to claim 13 or 14, wherein the system use state comprises the number of measurement data receptions from a measurement device of each system user.

16. The management device of the remote service providing system according to any one of claims 13 to 15, wherein the system use state comprises the number times of remote service provisions to each system user.

17. The management device of the remote service providing system according to claim 16, wherein the remote service comprises transmission of an instructive message from a doctor or an adviser via the communications line.

18. A charge computing method in a remote service providing system that, by connecting a measurement device owned by a system user and a service providing device of a service provider via a communications line, transmits measurement data to the service providing device via the communications line and provides a remote service from the service providing device to the system user via the communications line, the method comprising:
storing, in the service providing device, system use information comprising a system use state of each system user;
acquiring from the service providing device the system use information for each system user; and
computing the charge on the basis of the system use state included in the acquired system use information.

19. The charge computing method in the remote service providing system according to claim 18, wherein the system use state comprises the number of measurement data receptions from a measurement device of each system user.

20. The charge computing method in the remote service providing system according to claim 18 or 19, wherein the system use state comprises the number of provisions of a remote service to each system user.
